# EUROPÄISCHE PATENTSCHRIFT

(11) **EP 0 731 092 B1**
(45) Veröffentlichungstag und Bekanntmachung des Hinweises auf die Patenterteilung: **06.05.1998**
(21) Anmeldenummer: 96102651.5
(22) Anmeldetag: 22.02.1996
(51) Int. Cl.: C07D 209/86, C07D 209/88

(54) **Verfahren zur Herstellung von substituierten 1,2,3,4-Tetrahydrocarbazolen**
Process for producing substituted 1,2,3,4-tetrahydrocarbazoles
Procédé pour la préparation de dérivés substitués de la 1,2,3,4-tétrahydrocarbazoles

(30) Priorität: 06.03.1995 DE 19507751
(43) Veröffentlichungstag der Anmeldung: 11.09.1996
(73) Patentinhaber: BAYER AG, 51368 Leverkusen (DE)
(72) Erfinder: Landscheidt, Heinz, Dr., 47057 Duisburg (DE); Klausener, Alexander, Dr., 50670 Köln (DE); Zirngiebl, Eberhard, Dr., 51061 Köln (DE); Jentsch, Jörg-Dietrich, 45468 Mülheim a.d. Ruhr (DE)

(56) Entgegenhaltungen:
- EP-A- 0 179 217
- DE-A- 3 631 824
- US-A- 3 959 309
- US-A- 4 988 820

## Beschreibung

Die vorliegende Erfindung betrifft ein Verfahren zur Herstellung substituierter 1,2,3,4-Tetrahydrocarbazole durch heterogen katalysierte selektive Kernhydrierung substituierter Phenole und anschließende Umsetzung der so erhaltenen substituierten Cyclohexanone mit gegebenenfalls substituierten Phenylhydrazinen.

Substituierte 1,2,3,4-Tetrahydrocarbazole sind als Zwischenprodukte für die Herstellung von pharmazeutischen Wirkstoffen von Bedeutung. So wird in US 4.988.820 und DE 3.631.824 die Verwendung derartiger Verbindungen als Arzneimittel beschrieben.

Substituierte 1,2,3,4-Tetrahydrocarbazole lassen sich allgemein durch Umsetzung substituierter Cyclohexanone mit Phenylhydrazin und anschließende Cyclisierung der intermediär erhaltenen substituierten Phenylhydrazone in Gegenwart von Säure herstellen. Es handelt sich bei diesem Verfahren um eine spezielle Ausführungsform der Indol-Synthese nach Fischer. In US 3.959.309 wird beispielsweise die Reaktion von 4-Acetamido-cyclohexanon und Phenylhydrazin zu 3-Acetamido-1,2,3,4-tetrahydrocarbazol beschrieben.

Problematisch ist, daß die benötigten substituierten Cyclohexanone in der Regel nicht auf einfache Weise zugänglich sind, sondern durch aufwendige Oxidationsverfahren gewonnen werden müssen. Um keine Ausbeuteminderung bei den folgenden Umsetzungsschritten hinnehmen zu müssen und um mögliche Verunreinigungen der pharmazeutisch wirksamen Zielverbindungen mit den zur Herstellung der genannten substituierten Cyclohexanone verwendeten Oxidationsmittel, im allgemeinen Schwermetallverbindungen, zu vermeiden, müssen die so erhaltenen Cyclohexanone einer aufwendigen Reinigungsprozedur unterzogen werden, wodurch sich die Synthese der gewünschten substituierten 1,2,3,4-Tetrahydrocarbazole erheblich verteuert.

Es bestand daher die Aufgabe, ausgehend von leicht zugänglichen und möglichst bereits technisch verfügbaren Rohstoffen, ein vereinfachtes Verfahren zur Herstellung von substituierten 1,2,3,4-Tetrahydrocarbazolen zu finden.

Die Erfindung betrifft ein Verfahren zur Herstellung von substituierten 1,2,3,4-Tetrahydrocarbazolen der Formel 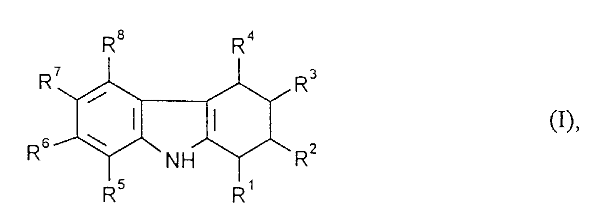 in der
- R¹, R², R³ und R⁴: unabhängig voneinander Wasserstoff, C₁-C₄-Alkyl, C₃-C₈-Cycloalkyl, Halogen, Hydroxy, C₁-C₄-Alkoxy, C₃-C₈-Cycloalkoxy, C₁-C₄-Alkylamino, N(C₁-C₄-Alkyl)₂-, NH-C₁-C₄-Acyl, COOH, -COOC₁-C₄-Alkyl oder -CH₂-Q bedeuten, wobei Q für Hydroxy, C₁-C₄-Alkoxy oder NH-C₁-C₄-Acyl steht und wobei mindestens einer von R¹ bis R⁴ von Wasserstoff verschieden ist, und in der
- R⁵, R⁶, R⁷ und R⁸: unabhängig voneinander Wasserstoff, C₁-C₄-Alkyl, Halogen oder C₁-C₄-Alkoxy bedeuten,
das dadurch gekennzeichnet ist, daß man ein substituiertes Phenol der Formel 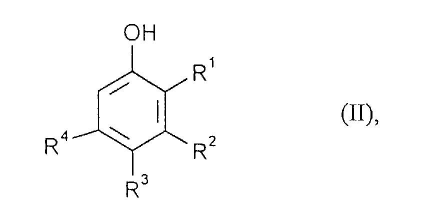 in der
- R¹, R², R³ und R⁴: die oben angegebene Bedeutung haben,
in Gegenwart eines gegebenenfalls auf einen Träger aufgebrachten Katalysators aus der Reihe der Metalle der Gruppe VIII B des Periodensystems der Elemente (Mendelejew) sowie gegebenenfalls in Gegenwart eines oder mehrerer weiterer Zusatzstoffe aus der Reihe der alkalisch reagierenden (Erd)Alkali- oder Ammoniumsalze in einem oder mehreren Ethern als Lösungsmittel katalytisch hydriert, die nach Abtrennen des Katalysators erhaltene Hydrierlösung mit einem Phenylhydrazin der Formel 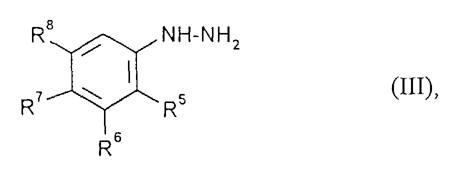 in der
- R⁵, R⁶, R⁷ und R⁸: die oben angegebene Bedeutung haben,
gegebenenfalls nach Zusatz von Wasser, zur Reaktion bringt und das dabei als Zwischenprodukt erhaltene substituierte Phenylhydrazon, bevorzugt nach Zwischenisolierung, einer intramolekularen Cyclisierung unter sauren Bedingungen unterzieht.

Halogen ist beispielsweise Fluor, Chlor, Brom, bevorzugt Chlor.

C₁-C₄-Alkyl ist beispielsweise Methyl, Ethyl, Propyl, Isopropyl, Butyl, Isobutyl, sec.-Butyl oder tert.-Butyl, bevorzugt Methyl oder Ethyl, besonders bevorzugt Methyl.

C₁-C₄-Alkoxy ist beispielsweise Methoxy, Ethoxy, Propoxy, Isopropoxy, Butoxy, Isobutoxy, sec.-Butoxy oder tert.-Butoxy, bevorzugt Methoxy oder Ethoxy, besonders bevorzugt Methoxy.

C₃-C₈-Cycloalkyl ist beispielsweise Cyclopropyl, Cyclobutyl, Cyclopentyl, Cyclohexyl, Cycloheptyl, Cyclooctyl, mono-, di-, tri- oder tetramethyl-substituiertes Cycloalkyl der genannten Art mit insgesamt bis zu 8 C-Atomen oder entsprechend ethyl-substituiertes Cycloalkyl, bevorzugt Cyclopropyl, Cyclopentyl, Cyclohexyl oder methyl- oder ethyl-substituierte Derivate hiervon.

C₃-C₈-Cycloalkoxy leitet sich vom genannten Cycloalkyl in analoger Weise ab wie Alkoxy vom Alkyl.

C₁-C₄-Acyl ist beispielsweise Formyl, Acetyl, Propionyl, n-Butyryl oder i-Butyryl, bevorzugt Acetyl.

In bevorzugter Weise wird ein Phenol der Formel 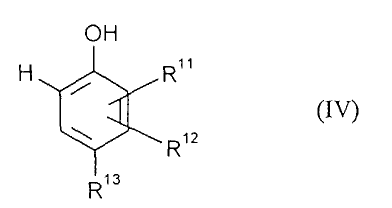 eingesetzt, in der
- R¹¹ und R¹²: unabhängig voneinander Wasserstoff, C₁-C₄-Alkyl, C₁-C₄-Alkoxy, Chlor oder Hydroxy bedeuten und
- R¹³: für Hydroxy, C₁-C₄-Alkyl, C₁-C₄-Alkoxy, C₁-C₄-Alkylamino, N(C₁-C₄-Alkyl)₂, NH-C₁-C₄-Acyl oder -COO-C₁-C₄-Alkyl steht.

In besonders bevorzugter Weise wird ein Phenol der Formel 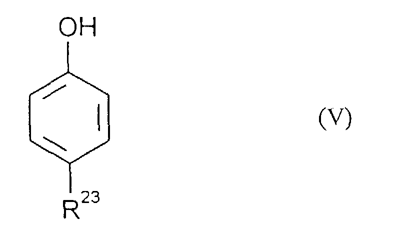 eingesetzt, in der
- R²³: Hydroxy, Methoxy, Ethoxy, Methylamino, Dimethylamino oder Acetamido bedeutet.

In weiterhin bevorzugter Form wird ein Phenylhydrazin der Formel 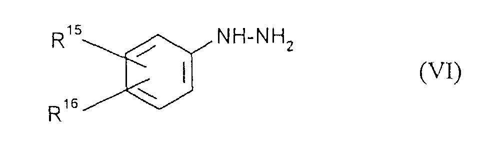 eingesetzt, in der
- R¹⁵ und R¹⁶: unabhängig voneinander Wasserstoff, Methyl, Methoxy oder Chlor bedeuten.

In besonders bevorzugter Weise wird nicht substituiertes Phenylhydrazin eingesetzt.

Zur Durchführung des erfindungsgemäßen Verfahrens wird das substituierte Phenol der Formel (II) in einem Lösungsmittel aus der Gruppe der Ether, bevorzugt in Diethylenglykoldimethylether, Ethylenglykoldimethylether, Dioxan und/oder Tetrahydrofuran, in einem Gewichtsverhältnis von 2:1 bis 1:10 (Phenol/Lösungsmittel), gegebenenfalls bei erhöhter Temperatur, gelöst bzw. suspendiert und mit einem gegebenenfalls auf einem festen Trägermaterial, wie Aktivkohle, Al₂O₃,SiO₂ u.a., aufgebrachten Katalysator aus der Gruppe der Metalle der Gruppe VIII B des Periodensystems der Elemente (Mendelejew) in einem Gewichtsverhältnis von 10.000:1 bis 10:1 (Phenol/Katalysator), sowie gegebenenfalls mit einem alkalisch reagierenden (Erd)Alkali- oder Ammoniumsalz in einem Gewichtsverhältnis von 20.000:1 bis 1 bis 20:1 (Phenol/Zusatzstoff), versetzt.

Metalle der Gruppe VIII B des Periodensystems sind beispielsweise Palladium, Ruthenium, Rhodium, Platin, Nickel, bevorzugt Palladium. In bevorzugter Weise wird Palladium auf einem Träger, besonders bevorzugt auf Kohle, insbesondere Aktivkohle, eingesetzt.

Als Zusatzstoffe einsetzbare alkalisch reagierende Salze sind beispielsweise die Hydroxide, Hydride, Carbonate, Hydrogencarbonate, Sulfite, Sulfide, Phosphate, Hydrogenphosphate, Boranate, Borate, C₁-C₆-Carboxylate von Li, Na, K, Rb, Cs, Mg, Ca, Sr, Ba, NH₄⁺ oder substituiertem NH₄⁺, bevorzugte die Carbonate, Hydrogencarbonate, Borate, Formiate und Acetate von Na, K, Ca, Mg, z.B. Natriumcarbonat und Borax.

Die erfindungsgemäße heterogen katalysierte Hydrierung erfolgt unter Rühren bei einer Temperatur von 20°C bis 250°C, bevorzugt 60 bis 230°C, besonders bevorzugt 100 bis 210°C, und einem Wasserstoffdruck von 1 bar bis 200 bar, bevorzugt 2 bis 150 bar, besonders bevorzugt 3 bis 100 bar. Bei der Hydrierung wird zur Erzielung der optimalen Selektivität die aulgenommene Wasserstoffmenge zweckmäßigerweise aufgezeichnet, um bei Erreichen der vorberechneten Wasserstoffmenge von 1,5 bis 2,5 Mol Wasserstoff je Mol Phenol die Hydrierung abbrechen zu können. Dies kann durch Erniedrigung der Rührerdrehzahl, Absenken der Temperatur und/oder Unterbrechung der Wasserstoffzufuhr erreicht werden.

Nach Beendigung der erfindungsgemäßen Hydrierung wird der Katalysator mit Hilfe üblicher Techniken, beispielsweise durch Filtration, abgetrennt.

Im weiteren Verlauf des erfindungsgemäßen Verfahrens wird die Reaktionsmischung, gegebenenfalls unter Zusatz von 50-2000 ml, bevorzugt 100 - 1000 ml Wasser je Mol eingesetztes Phenol, mit 0,5 bis 1,5 Mol des gegebenenfalls substituierten Phenylhydrazins der allgemeinen Formel (III) pro Mol eingesetztem Phenol versetzt und das sich dabei bildende und praktisch vollständig ausfallende substituierte Phenylhydrazon nach einer Reaktionszeit von etwa 1 bis 48 Stunden abfiltriert.

Durch Erhitzen des genannten intermediär anfallenden substituierten Phenylhydrazons in Gegenwart von Säure, bevorzugt in Gegenwart von gegebenenfalls mit Wasser verdünnter Säure, wird das gewünschte substituierte 1,2,3,4-Tetrahydrocarbazol der allgemeinen Formel (I) erhalten, das nach dem Abkühlen des Reaktionsgemisches ausfällt und in reiner Form, beispielsweise durch Filtration isoliert werden kann.

Bei der zur Durchführung des erfindungsgemäßen Verfahrens verwendeten Säuren handelt es sich um Mineralsäuren, wie HCI, H₂SO₄ oder H₃PO₄, oder um Alkancarbonsäuren mit 1 - 6 C-Atomen, bevorzugt um Alkancarbonsäuren mit 1 bis 3 C-Atomen, besonders bevorzugt um Essigsäure. Die Menge der Säure beträgt 0,5 - 20 Mol, bevorzugt 1 - 10 Äquivalente je Mol eingesetztes Phenol. Das Verdünnungswasser beträgt 1 - 100 ml je Äquivalent Säure.

### Beispiele

### Beispiel 1

Ein Gemisch aus 150 g Hydrochinon und 150 ml Diethylenglykoldimethylether wurde in Gegenwart von 3 g Pd (5 Gew.-%) auf Aktivkohle und unter Zusatz von 0,5 g Borax bei 160°C und einem Druck von 10 bar hydriert. Nach Aufnahme von 90 l Wasserstoff wurde die Hydrierung unterbrochen, der Katalysator abfiltriert und die Lösung nach Zusatz von 500 ml Wasser unter Rühren mit 120 g Phenylhydrazin versetzt. Man ließ 24 h lang bei Raumtemperatur nachreagieren, filtrierte das Hydrazon ab und wusch mit Wasser nach. Das so erhaltene Hydrazon wurde ohne weitere Reinigung mit 300 ml 40 %iger wäßriger Essigsäure 2 h lang unter Rückfluß erhitzt. Nach Abkühlen auf Raumtemperatur ließ man weitere 0,5 h lang bei 10°C stehen und saugte das ausgefallene Produkt ab. Man erhielt auf diese Weise 160 g 3-Hydroxy-1,2,3,4-tetrahydrocarbazol (Schmp. 140°C).

### Beispiel 2

Ein Gemisch aus 150 g 4-Methoxy-phenol und 150 ml Diethylenglykoldimethylether wurde in Gegenwart von 3 g Pd (5 Gew.-%) auf Aktivkohle und unter Zusatz von 0,5 g Borax bei 160°C und einem Druck von 10 bar hydriert. Nach Aufnahme von 90 l Wasserstoff wurde die Hydrierung unterbrochen, der Katalysator abfiltriert und die Lösung nach Zusatz von 500 g Wasser unter Rühren mit 110 g Phenylhydrazin versetzt. Man ließ 24 h lang bei Raumtemperatur nachreagieren, filtrierte das Hydrazon ab und wusch mit Wasser nach. Das so erhaltene Hydrazon wurde ohne weitere Reinigung mit 300 ml 40 %iger wäßriger Essigsäure 2 h lang unter Rückfluß erhitzt. Nach Abkühlen auf Raumtemperatur ließ man weitere 0,5 h lang bei 10°C stehen und saugte das ausgefallene Produkt ab. Man erhielt auf diese Weise 160 g 3-Methoxy-1,2,3,4-tetrahydrocarbazol (Smp.: 105°C).

### Beispiel 3

Ein Gemisch aus 150 g 4-Acetamido-phenol und 150 ml Diethylenglykoldimethylether wurde in Gegenwart von 3 g Pd (5 Gew.-%) auf Aktivkohle und unter Zusatz von 0,5 g Borax bei 160°C und einem Druck von 10 bar hydriert. Nach Aufnahme von 90 l Wasserstoff wurde die Hydrierung unterbrochen, der Katalysator abfiltriert und die Lösung nach Zusatz von 500 ml Wasser unter Rühren mit 120 g Phenylhydrazin versetzt. Man ließ 24 h lang bei Raumtemperatur nachreagieren, filtrierte das Hydrazon ab und wusch mit Wasser nach. Das so erhaltene Hydrazon wurde ohne weitere Reinigung mit 300 ml 40 %iger wäßriger Essigsäure 2 h lang unter Rückfluß erhitzt. Nach Abkühlen auf Raumtemperatur ließ man weitere 0,5 h lang bei 10°C stehen und saugte das ausgefallene Produkt ab. Man erhielt auf diese Weise 150 g 3-Acetamido-1,2,3,4-tetrahydrocarbazol (Schmp. 170°C).

## Patentansprüche

1. Verfahren zur Herstellung von substituierten 1,2,3,4-Tetrahydrocarbazolen der Formel 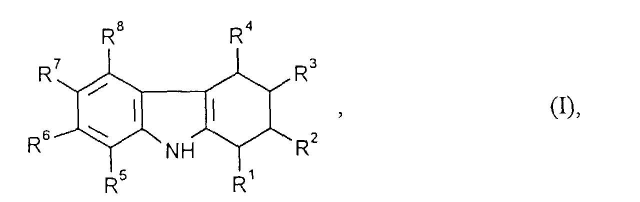 in der
R¹, R², R³ und R⁴ unabhängig voneinander Wasserstoff, C₁-C₄-Alkyl, C₃C₈-Cycloalkyl, Halogen, Hydroxy, C₁-C₄-Alkoxy, C₃-C₈-Cycloalkoxy, C₁-C₄-Alkylamino, N(C₁-C₄-Alkyl)₂, NH-C₁-C₄-Acyl, COOH, COOC₁-C₄-Alkyl oder -CH₂-Q bedeuten, wobei Q für Hydroxy, C₁-C₄-Alkoxy oder NH-C₁-C₄-Acyl steht und wobei mindestens einer von R¹ bis R⁴ von Wasserstoff verschieden ist, und in der
R⁵, R⁶, R⁷ und R⁸ unabhängig voneinander Wasserstoff, C₁-C₄-Alkyl, Halogen oder C₁-C₄-Alkoxy bedeuten,
dadurch gekennzeichnet, daß man ein substituiertes Phenol der Formel 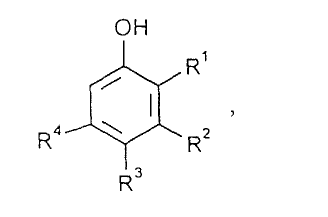 in der
R¹, R², R³ und R⁴ die oben angegebene Bedeutung haben,
in Gegenwart eines gegebenenfalls auf einen Träger aufgebrachten Katalysators aus der Reihe der Metalle der Gruppe VIII B des Periodensystems der Elemente (Mendelejew), sowie gegebenenfalls in Gegenwart eines oder mehrerer weiterer Zusatzstoffe aus der Reihe der alkalisch reagierenden (Erd)Alkali- oder Ammoniumsalze in einem oder mehreren Ethern als Lösungsmittel katalytisch hydriert, die nach Abtrennen des Katalysators erhaltene Hydrierlösung mit einem Phenylhydrazin der Formel 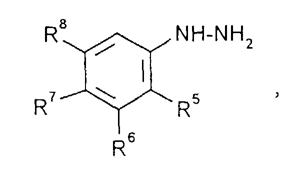 in der
R⁵, R⁶, R⁷ und R⁸ die oben angegebene Bedeutung haben,
gegebenenfalls nach Zusatz von Wasser, zur Reaktion bringt und das dabei als Zwischenprodukt erhaltene substituierte Phenylhydrazon, bevorzugt nach Zwischenisolierung, einer intramolekularen Cyclisierung unter sauren Bedingungen unterzieht.

2. Verfahren nach Anspruch 1, dadurch gekennzeichnet, daß die Hydrierung in Diethylenglykoldimethylether, Ethylenglykoldimethylether, 1,4-Dioxan, Tetrahydrofuran oder einem Gemisch mehrerer von ihnen durchgeführt wird.

3. Verfahren nach Anspruch 1, dadurch gekennzeichnet, daß als substituiertes Phenol Hydrochinon, 4-Methoxy-phenol oder p-Acetylamino-phenol eingesetzt wird.

4. Verfahren nach Anspruch 1, dadurch gekennzeichnet, daß die Hydrierung in einem Temperaturbereich von 20°C bis 250°C, bevorzugt von 60°C bis 230°C, besonders bevorzugt von 100°C bis 210°C, durchgeführt wird.

5. Verfahren nach Anspruch 1, dadurch gekennzeichnet, daß die Hydrierung in einem Druckbereich von 1 bar bis 200 bar, bevorzugt von 2 bar bis 150 bar, besonders bevorzugt von 3 bar bis 100 bar, durchgeführt wird.

6. Verfahren nach Anspruch 1, dadurch gekennzeichnet, daß als Katalysator Palladium auf einem Träger, bevorzugt auf Kohle, eingesetzt wird.

7. Verfahren nach Anspruch 1, dadurch gekennzeichnet, daß als Zusatzstoff für die Hydrierung Natriumcarbonat oder Borax verwendet wird.

8. Verfahren nach Anspruch 1, dadurch gekennzeichnet, daß nicht substituiertes Phenylhydrazin eingesetzt wird.

9. Verfahren nach Anspruch 1, dadurch gekennzeichnet, daß zur Durchführung des Cyclisierungsschrittes als Säure eine wäßrige Alkancarbonsäure mit 1 bis 3 C-Atomen, bevorzugt Essigsäure, im Gemisch mit Wasser eingesetzt wird.

## Claims

1. Process for the preparation of substituted 1,2,3,4-tetrahydrocarbazoles of the formula 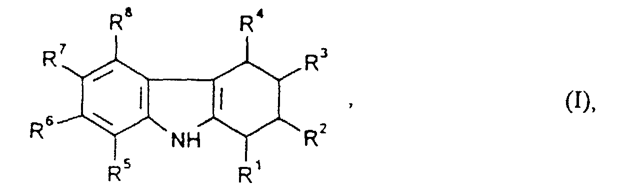 in which
R¹, R², R³ and R⁴, independently of one another, are hydrogen, C₁-C₄-alkyl, C₃-C₈-cycloalkyl, halogen, hydroxyl, C₁-C₄-alkoxy, C₃-C₈-cycloalkoxy, C₁-C₄-alkylamino, N(C₁-C₄-alkyl)₂, NH-C₁-C₄-acyl, COOH, COOC₁-C₄-alkyl or -CH₂-Q, where Q is hydroxyl, C₁-C₄-alkoxy or NH-C₁-C₄-acyl and where at least one of R¹ to R⁴ is not hydrogen, and in which
R⁵, R⁶, R⁷ and R⁸, independently of one another, are hydrogen, C₁-C₄-alkyl, halogen or C₁-C₄-alkoxy,
characterized in that a substituted phenol of the formula 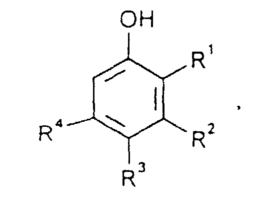 in which
R¹, R², R³ and R⁴ are as defined above,
is catalytically hydrogenated in the presence of a supported or unsupported catalyst selected from the series of metals from group VIII B of the Periodic Table of the Elements (Mendeleyev), and also optionally in the presence of one or more further additives selected from the series consisting of alkaline alkali(ne earth) metal salts or ammonium salts in one or more ethers as solvent, the hydrogenation solution obtained after removal of the catalyst, optionally after the addition of water, is reacted with a phenylhydrazine of the formula 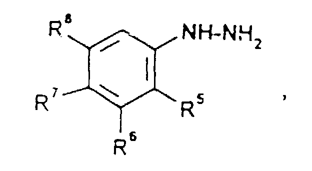 in which
R⁵, R⁶, R⁷ and R⁸ are as defined above,
and the resulting substituted phenylhydrazone which is obtained as intermediate is, preferably after isolation, subjected to an intramolecular cyclization under acid conditions.

2. Process according to Claim 1, characterized in that the hydrogenation is carried out in diethylene glycol dimethyl ether, ethylene glycol dimethyl ether, 1,4-dioxane, tetrahydrofuran or a mixture of two or more of these.

3. Process according to Claim 1, characterized in that the substituted phenol used is hydroquinone, 4-methoxy-phenol or p-acetylamino-phenol.

4. Process according to Claim 1, characterized in that the hydrogenation is carried out in a temperature range of from 20°C to 250°C, preferably from 60°C to 230°C, particularly preferably from 100°C to 210°C.

5. Process according to Claim 1, characterized in that the hydrogenation is carried out in a pressure range of from 1 bar to 200 bar, preferably from 2 bar to 150 bar, particularly preferably from 3 bar to 100 bar.

6. Process according to Claim 1, characterized in that the catalyst used is palladium on a support, preferably on carbon.

7. Process according to Claim 1, characterized in that the additive used for the hydrogenation is sodium carbonate or borax.

8. Process according to Claim 1, characterized in that unsubstituted phenylhydrazine is used.

9. Process according to Claim 1, characterized in that the acid used to carry out the cyclization step is an aqueous alkanecarboxylic acid having from 1 to 3 carbon atoms, preferably acetic acid, as a mixture with water.

## Revendications

1. Procédé pour la préparation de 1,2,3,4-tétrahydrocarbazoles substitués répondant à la formule 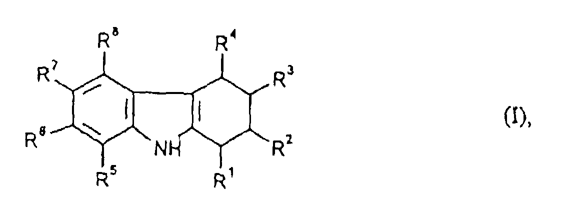 dans laquelle
R¹, R², R³ et R⁴ représentent, indépendamment l'un de l'autre, un atome d'hydrogène, un groupe alkyle en C₁-C₄, un groupe cycloalkyle en C₃-C₈, un atome d'halogène, un groupe hydroxyle, un groupe alcoxy en C₁-C₄, un groupe cycloalcoxy en C₃-C₈, un groupe alkyl(en C₁-C₄)amino, un groupe N(alkyle en C₁-C₄)₂, un groupe NH-acyle en C₁-C₄, un groupe COOH, un groupe COO-alkyle en C₁-C₄ ou un groupe -CH₂-Q où Q représente un groupe hydroxyle, un groupe alcoxy en C₁-C₄ ou un groupe NH-acyle en C₁-C₄, et dans laquelle au moins un des radicaux R₁ à R₄ représente autre chose qu'un atome d'hydrogène, et dans laquelle
R⁵, R⁶, R⁷ et R⁸ représentent, indépendamment l'un de l'autre, un atome d'hydrogène, un groupe alkyle en C₁-C₄, un atome d'halogène ou un groupe alcoxy en C₁-C₄,
caractérisé en ce qu'on soumet à une hydrogénation catalytique dans un ou plusieurs éthers à titre de solvant, un phénol substitué répondant à la formule 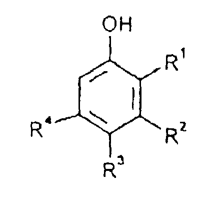 dans laquelle
R¹, R², R³ et R⁴ ont la signification indiquée ci-dessus,
en présence d'un catalyseur éventuellement déposé sur un support, choisi parmi la série des métaux du groupe VIII B du tableau périodique des éléments (Mendelejew) et éventuellement en présence d'un ou plusieurs autres additifs choisis parmi la série comprenant les sels de métaux alcalins (alcalino-terreux) ou d'ammonium réagissant de manière alcaline, on amène la solution hydrogénée obtenue après séparation du catalyseur à réagir avec une phénylhydrazine répondant à la formule 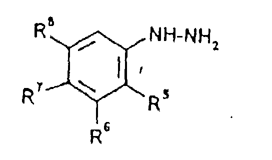 dans laquelle
R⁵, R⁶, R⁷ et R⁸ ont la signification indiquée ci-dessus,
éventuellement après addition d'eau, et on soumet la phénylhydrazone substituée, obtenue en l'occurrence sous forme de produit intermédiaire, de préférence après isolation intermédiaire, à une cyclisation intramoléculaire dans des conditions acides.

2. Procédé selon la revendication 1, caractérisé en ce qu'on effectue l'hydrogénation dans de l'éther diméthylique de diéthylèneglycol, dans de l'éther diméthylique d'éthylèneglycol, dans du 1,4-dioxanne, dans du tétrahydrofuranne ou dans un mélange de plusieurs d'entre eux.

3. Procédé selon la revendication 1, caractérisé en ce qu'on met en oeuvre, à titre de phénol substitué, de l'hydroquinone, le 4-méthoxyphénol ou le p-acétylaminophénol.

4. Procédé selon la revendication 1, caractérisé en ce qu'on effectue l'hydrogénation dans un domaine de température de 20°C à 250°C, de préférence de 60°C à 230°C, de manière particulièrement préférée de 100°C à 210°C.

5. Procédé selon la revendication 1, caractérisé en ce qu'on effectue l'hydrogénation dans un domaine de pression de 1 bar à 200 bar, de préférence de 2 bar à 150 bar, de manière particulièrement préférée de 3 bar à 100 bar.

6. Procédé selon la revendication 1, caractérisé en ce qu'on met en oeuvre, à titre de catalyseur, du palladium déposé sur un support, de préférence sur du charbon.

7. Procédé selon la revendication 1, caractérisé en ce qu'on utilise, comme additif pour l'hydrogénation, du carbonate de sodium ou du borax.

8. Procédé selon la revendication 1, caractérisé en ce qu'on met en oeuvre de la phénylhydrazine non substituée.

9. Procédé selon la revendication 1, caractérisé en ce qu'on met en oeuvre, pour la réalisation de l'étape de cyclisation, à titre d'acide, un acide alcanecarboxylique aqueux contenant de 1 à 3 atomes de carbone, de préférence l'acide acétique en mélange avec de l'eau.
